# EUROPEAN PATENT SPECIFICATION

(11) **EP 2 547 351 B1**
(45) Date of publication and mention of the grant of the patent: **27.04.2016**
(21) Application number: 11756786.7
(22) Date of filing: 14.03.2011
(51) Int. Cl.: A61K 38/08, A61K 38/04, A61K 9/00

(54) **PHARMACEUTICAL COMPOSITIONS OF GROWTH HORMONE SECRETAGOGUE RECEPTOR LIGANDS**
PHARMAZEUTISCHE ZUSAMMENSETZUNGEN AUS WACHSTUMSHORMON-SEKRETAGOGEN-REZEPTORLIGANDEN
COMPOSITIONS PHARMACEUTIQUES CONTENANT DES LIGANDS DE RÉCEPTEURS SÉCRÉTAGOGUES D'HORMONE DE CROISSANCE

(30) Priority: 15.03.2010 US 340290 P
(43) Date of publication of application: 23.01.2013
(73) Proprietor: IPSEN PHARMA S.A.S., 92100 Boulogne-Billancourt (FR)
(72) Inventor: DONG, Zheng, Xin, Holliston MA 01746 (US); ZHANG, Jundong, Newton MA 02461 (US)
(74) Representative: Wells, Andrew
(86) International application number: PCT/US2011/028283
(87) International publication number: WO 2011/115871

(56) References cited:
- WO-A1-99/44642
- WO-A1-2010/016936
- WO-A1-2011/117851
- WO-A2-02/17918
- WO-A2-2007/084460
- WO-A2-2008/041245
- US-A1- 2005 148 515
- US-A1- 2006 039 862
- US-A1- 2008 171 700
- BOWERS C Y ET AL: "IN-VITRO AND IN-VIVO ACTIVITY OF A NEW SYNTHETIC HEXA PEPTIDE THAT ACTS ON THE PITUITARY TO SPECIFICALLY RELEASE GROWTH HORMONE", ENDOCRINOLOGY, vol. 114, no. 5, 1984, pages 1537-1545, XP009171336, ISSN: 0013-7227

## Description

### BACKGROUND OF THE INVENTION

The present invention relates to improvements in compositions containing peptides that are ligands of the growth hormone secretagogue (GHS) receptor, or pharmaceutically acceptable salts thereof, methods for preparing such compositions, and methods of using such compositions to treat mammals. In particular, the present invention relates to a pharmaceutical composition comprising a pamoate salt of H-Inp-D-Bal-D-Tip-Phe-Apc-NH₂, which is a ligand of the GHS receptor, and in which, after subcutaneous or intramuscular administration to a subject, the peptide forms an *in situ* depot at physiological pH that is slowly dissolved and released into the body fluid and bloodstream. The present composition of the invention further comprises polyethylene glycol. The polyethylene glycol may have an average molecular weight of lower than 1000.

The pulsatile release of growth hormone from the pituitary somatotrops is regulated by two hypothalamic neuropeptides: growth hormone-releasing hormone and somatostatin. Growth hormone-releasing hormone stimulates release of growth hormone, whereas, somatostatin inhibits secretion of growth hormone. (Frohman et al., Endocr. Rev. 1986, 7, 223-253, and Strobi et al., Pharmacol. Rev. 1994, 46, 1-34.)

Release of growth hormone from the pituitary somatotrops can also be controlled by growth hormone-releasing peptides (GHRP's). A hexapeptide, His-D-Trp-Ala-Trp-D-Phe-Lys-amide (GHRP-6), was found to release growth hormone from somatotrops in a dose-dependent manner in several species including man. (Bowers et al., Endocrinology 1984, 114, 1537-1545.) Subsequent chemical studies on GHRP-6 led to the identification of other potent growth hormone secretagogues such as GHRP-I, GHRP-2 and hexarelin (Cheng et al., Endocrinology 1989, 124, 2791-2798, Bowers, C. Y. Novel GH-Releasing Peptides. In: Molecular and Clinical Advances in Pituitary Disorders. Ed: Melmed, S.; Endocrine Research and Education, Inc., Los Angeles, CA, USA 1993, 153-157, and Deghenghi et al.. Life Sci. 1994, 54, 1321-1328):
GHRP-I Ala-His-D-(2')-Nal-Ala-Trp-D-Phe-Lys-NH₂;
GHRP-2 D-Ala-D-(2')-Nal-Ala-Trp-D-Nal-Lys-NH₂;
Hexarelin His-D-2-MeTip-Ala-Trp-D-Phe-Lys-NH₂.
GHRP-I, GHRP-2, GHRP-6, and hexarelin are synthetic growth hormone secretagogues (GHS's). GHS's stimulate secretion of growth hormone by a mechanism different from that of growth hormone-releasing hormone. (Bowers et al.. Endocrinology 1984, 114, 1537-1545, Cheng et al. Endocrinology 1989, 124, 2791-2798, Bowers, C. Y. Novel GH-Releasing Peptides. In: Molecular and Clinical Advances in Pituitary Disorders. Ed: Melmed, S.; Endocrine Research and Education, Inc., Los Angeles, CA, USA 1993, 153-157, and Deghenghi et al., Life Sci. 1994, 54, 1321-1328.)

The low oral bioavailability (<1%) of the peptidyl growth hormone secretagogues stimulated search for non-peptide compounds mimicking action of GHRP-6 in the pituitary. Several benzolactams and spiroindanes have been reported to stimulate growth hormone release in various animal species and in man. (Smith et al.. Science 1993, 260, 1640-1643, Patchett et al, Proc. Natl Acad. Sci. USA. 1995, 92, 7001-7005, and Chen et al., Bioorg. Mod. Chem. Lett. 1996, 6, 2163-2169.) A specific example of a small spiroindane is MK-0677 (Patchett et al. Proc. Natl. Acad. Sci. USA. 1995, 92, 7001-7005):

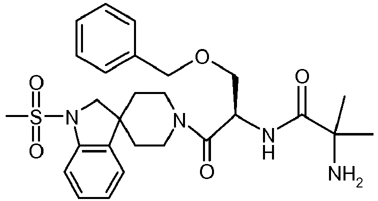

The actions of the above-mentioned GHS's (both peptide and non-peptide) appear to be mediated by a specific growth hormone secretagogue receptor (GHS receptor). (Howard et al, Science 1996, 273, 974-977, and Pong et al, Molecular Endocrinology 1996, 10, 57-61.) This receptor is present in the pituitary and hypothalamus of various mammalian species (GHSR1a) and is distinct from the growth hormone-releasing hormone (GHRH) receptor. The GHS receptor was also detected in the other areas of the central nervous system and in peripheral tissues, for instance adrenal and thyroid glands, heart, lung, kidney and skeletal muscles. (Chen et al, Bioorg. Med. Chem. Lett. 1996, 6, 2163-2169, Howard et al, Science 1996,273, 974-977, Pong et al, Molecular Endocrinology 1996, 10, 57-61, Guan et al, Mol. Brain Res. 1997, 48, 23-29, and McKee et al, Genomics 1997, 46, 426-434.) A truncated version of GHSR1a has been reported. (Howard et al., Science 1996, 273, 974-977.)

The GHS receptor is a G-protein coupled-receptor. Effects of GHS receptor activation includes depolarization and inhibition of potassium channels, an increase in intercellular concentrations of inositol triphosphate (IP3), and a transient increase in the concentrations of intracellular calcium. (Pong et al., Molecular Endocrinology 1996,10, 57-61*,* Guan et al., Mol. Brain Res. 1997, 48, 23-29, and McKee et al., Genomics 1997, 46,426-434.)

Ghrelin is a naturally occurring peptide which is believed to be an endogenous ligand for the GHS receptor. (Kojima et al., Nature 1999, 402, 656-660.) The native structures of ghrelins from several mammalian and non-mammalian species of animals are known. (Kaiya et al., J. Biol. Chem. 2001, 276, 40441-40448; International Patent Application PCT/JP00/04907 (WO 01/07475).) A core region present in ghrelin was found to provide for activity at the GHS receptor. The core region comprises the four N-terminal amino acids, where the serine at position 3 is normally modified with n-octanoic acid. In addition to acylation by n-octanoic acid native ghrelin also has been observed to be acylated with n-decanoic acid. (Kaiya et al., J. Biol. Chem. 2001, 276, 40441-40448.) Ghrelin analogs have a variety of different therapeutic uses as well as uses as research tools.

### SUMMARY OF THE INVENTION

The present disclosure provides a formulation of a pharmaceutical composition comprising a pamoate salt of a peptide that acts as a ligand of the GHS receptor. Particularly the peptide is the following peptide which is referred to hereinafter as "Example 1": H-Inp-D-Bal-D-Trp-Phe-Apc-NH₂, which is a ligand of the GHS receptor, wherein, after subcutaneous or intramuscular administration to a subject, the peptide forms a precipitate at physiological pH that is slowly dissolved and released into the body fluid and bloodstream, thereby resulting in attenuated side effects and improved efficacy.

Accordingly, the invention provides a pharmaceutical composition of a clear solution, comprising a peptide that acts as a ligand of the GHS receptor, wherein the peptide is H-Inp-D-Bal-D-Trp-Phe-Apc-NH₂, or a pharmaceutically acceptable salt thereof, in which the peptide forms an *in situ* depot after subcutaneous or intramuscular administration to a subject, wherein the pharmaceutical composition further comprises PEG, wherein said peptide is in a pamoate salt form, and wherein said clear solution is an aqueous solution. The invention is further defined in the claims.

The disclosure herein may be summarized in the following paragraphs below, as well as in the claims.
(1) In one aspect, the present disclosure is directed to a pharmaceutical composition of a clear solution, a gel or a semi-solid, or a suspension, comprising a peptide that acts as a ligand of the GHS receptor, or a pharmaceutically acceptable salt
   thereof, in which the peptide forms a precipitate after subcutaneous or intramuscular administration to a subject.
(2) The pharmaceutical composition according to paragraph 1, wherein said peptide in said clear solution precipitates *in vivo* to form an *in situ* depot that is slowly dissolved and released into the body fluid and bloodstream, and wherein said clear solution is a purely aqueous solution, a purely organic solution, or an aqueous solution having an organic component.
(3) The pharmaceutical composition according to paragraph 1 or paragraph 2, wherein said peptide is Example 1, *i.e*., H-Inp-D-Bal-D-Trp-Phe-Apc-NH₂.
(4) The pharmaceutical composition according to any one of the preceding paragraphs, wherein said peptide is in a pamoate salt form.
(5) The pharmaceutical composition according to any one of the preceding paragraphs, further comprising an organic component which increases the solubility of said peptide in an aqueous solution or decreases the viscosity of a gel or a semi-solid.
(6) The pharmaceutical composition according to paragraph 5, wherein said organic component is an organic polymer, an alcohol, DMSO, DMF, or dimethylacetamide (DMA).
(7) The pharmaceutical composition according to paragraph 6, wherein said organic polymer is PEG.
(8) The pharmaceutical composition according to paragraph 7, wherein said PEG has an average molecular weight of from about 200 to about 10,000.
(9) The pharmaceutical composition according to paragraph 8, wherein said peptide is dissolved in a PEG200 or PEG400 aqueous solution, in which the volume-to-volume ratio of PEG to water is from about 1:99 to about 99:1.
(10) The pharmaceutical composition according to paragraph 9, wherein said peptide is dissolved in a PEG200 or PEG400 aqueous solution, in which the volume-to-volume ratio of PEG to water is from about 1:9 to about 1:1.
(11) The pharmaceutical composition according to paragraph 6, wherein said alcohol is ethanol or isopropyl alcohol.
(12) The pharmaceutical composition according to any one of the preceding paragraphs, wherein the weight-to-volume concentration of said peptide is between about 0.1 mg/mL and about 600 mg/mL.
(13) The pharmaceutical composition according to any one of the preceding paragraphs, wherein the pH of said composition is between about 3.0 and about 8.0.
(14) The pharmaceutical composition according to paragraph 13, wherein said pamoate salt of H-Inp-D-Bal-D-Trp-Phe-Apc-NH₂ is dissolved in a PEG400/aqueous solution, in which the volume-to-volume ratio of PEG400 to water is about 1:1, and in which the weight-to-volume concentration of the peptide is about 200 mg/mL.
(15) The pharmaceutical composition according to paragraph 13, wherein said pamoate salt of H-Inp-D-Bal-D-Trp-Phe-Apc-NH₂ is dissolved in a PEG200/aqueous solution, in which the volume-to-volume ratio of PEG200 to water is about 1:1, and in which the weight-to-volume concentration of the peptide is about 200 mg/mL.
(16) The pharmaceutical composition according to paragraph 13, wherein said pamoate salt of H-Inp-D-Bal-D-Trp-Phe-Apc-NH₂ is dissolved in a PEG400/PBS solution, in which the volume-to-volume ratio of PEG400 to PBS is about 1:1, and in which the weight-to-volume concentration of the peptide is about 300 mg/mL.
(17) The pharmaceutical composition according to paragraph 13, wherein said pamoate salt of H-Inp-D-Bal-D-Trp-Phe-Apc-NH₂ is dissolved in a PEG400/saline solution, in which the volume-to-volume ratio of PEG400 to saline solution is about 1:1, and in which the weight-to-volume concentration of the peptide is about 300 mg/mL.
(18) The pharmaceutical composition according to any one of the preceding paragraphs, further comprising a preservative.
(19) The pharmaceutical composition according to paragraph 18, wherein said preservative is selected from the group consisting of m-cresol, phenol, benzyl alcohol, and methyl paraben.
(20) The pharmaceutical composition according to paragraph 19, wherein said preservative is present in a concentration from about 0.01 mg/mL to about 100 mg/mL.
(21) The pharmaceutical composition according to any one of the preceding paragraphs, further comprising an isotonic agent.
(22) The pharmaceutical composition according to paragraph 21, wherein said isotonic agent is present in a concentration from about 0.01 mg/mL to about 100 mg/mL.
   (1) The pharmaceutical composition according to any one of the preceding paragraphs, further comprising a stabilizer.
   (2) The pharmaceutical composition according to paragraph 23, wherein said stabilizer is selected from the group consisting of imidazole, arginine and histidine.
   (3) The pharmaceutical composition according to any one of the preceding paragraphs, further comprising a surfactant.
   (4) The pharmaceutical composition according to any one of the preceding paragraphs, further comprising a chelating agent.
   (5) The pharmaceutical composition according to any one of the preceding paragraphs, further comprising a buffer.
   (6) The pharmaceutical composition according to paragraph 27, wherein said buffer is selected from the group consisting of Tris, ammonium acetate, sodium acetate, glycine, aspartic acid, and Bis-Tris.
   (7) The pharmaceutical composition according to any one of the preceding paragraphs, further comprising a divalent metal.
   (8) The pharmaceutical composition according to paragraph 29, wherein said divalent metal is zinc.

The following compounds may also be advantageously employed to constitute the pharmaceutical compositions of the present disclosure:
Example 2: H-Inp-D-INal-D-Trp-3Pal-Lys-NH₂;
Example 3: H-Inp-D-2Nal-D-Trp-4Pal-Lys-NH₂;
Example 4: H-Inp-D-2Nal-D-Trp-Orn-Lys-NH₂;
Example 5: H-Inp-D-Bip-D-Trp-Phe-Lys-NH₂;
Example 6: H-Inp-D-2Nal-D-Trp-Thr(Bzl)-Lys-NH₂;
Example 7: H-Inp-D-2Nal-D-Trp-Pff-Lys-NH₂;
Example 8: H-Inp-D-2Nal-D-Trp-2Thi-Lys-NH₂;
Example 9: H-Inp-D-2Nal-D-Trp-Taz-Lys-NH₂;
Example 10: H-Inp-D-Dip-D-Trp-Phe-Lys-NH₂;
Example 11: H-Inp-D-Bpa-D-Trp-Phe-Lys-NH₂;
Example 12: H-Inp-D-2Nal-D-Bpa-Phe-Lys-NH₂;
Example 13: H-Inp-D-2Nal-D-Trp-3Pal-NH₂;
Example 14: H-Inp-D-2Nal-D-Trp-4Pal-NH₂;
Example 15: H-Inp-D-1Nal-D-Trp-3Pal-NH₂;
Example 16: H-Inp-D-Bip-D-Trp-Phe-NH₂;
Example 17: H-Inp-D-2Nal-D-Trp-Thr(Bzl)-NH₂;
Example 18: H-Inp-D-2Nal-D-Trp-Pff-NH₂;
Example 19: H-Inp-D-2Nal-D-Trp-2Thi-NH₂;
Example 20: H-Inp-D-2Nal-D-Trp-Taz-NH₂;
Example 21: H-Inp-D-Dip-D-Trp-Phe-NH₂;
Example 22: H-Inp-D-2Nal-D-Dip-Phe-NH₂;
Example 23: H-Inp-D-Bal-D-Trp-Phe-NH₂;
Example 24: H-Inp-D-2Nal-D-Bal-Phe-NH₂;
Example 25: H-Inp-D-2Nal-D-Trp-3Pal-Lys-NH₂;
Example 26: H-Inp-D-Bal-D-Trp-2Thi-Lys-NH₂;
Example 27: H-Inp-D-Bal-D-Trp-Phe-Lys-NH₂;
Example 28: H-Inp-D-1Nal-D-Trp-2Thi-Lys-NH₂;
Example 29: H-Inp-D-2Nal-D-Trp-Phe-Apc-NH₂;
Example 30: H-Inp-D-1Nal-D-Trp-Phe-Apc-NH₂;
Example 31: H-Apc-D-2Nal-D-Trp-Phe-Lys-NH₂;
Example 32: H-Apc-D-1Na1-D-Trp-2Thi-Lys-NH₂;
Example 33: H-Inp-D-lNal-D-Trp-2Thi-NH₂;
Example 34: H-Apc-D-1Nal-D-Trp-Phe-NH₂;
Example 35: H-Inp-D-INal-D-Trp-Taz-Lys-NH₂;
Example 36: H-Inp-D-Bal-D-Trp-Taz-Lys-NH₂;
Example 37: H-Apc-D-1Nal-D-Trp-Taz-Lys-NH₂;
Example 38: H-Apc-D-Bal-D-Trp-Taz-Lys-NH₂;
Example 39: H-Apc-D-Bal-D-Trp-2Thi-Lys-NH₂;
Example 40: H-Inp-D-1Na1-D-Trp-2Thi-Apc-NH₂;
Example 41: H-Inp-D-Bal-D-Trp-2Thi-Apc-NH₂;
Example 42: H-Apc-D-1Nal-D-Trp-2Thi-Apc-NH₂;
Example 43: H-Apc-D-Bal-D-Trp-2Thi-Apc-NH₂;
Example 44: H-Apc-D-1Nal-D-Trp-Phe-Lys-NH₂;
Example 45: H-Apc-D-Bal-D-Trp-Phe-Lys-NH₂;
Example 46: H-Apc-D-1Nal-D-Trp-Phe-Apc-NH₂;
Example 47: H-Apc-D-Bal-D-Trp-Phe-Apc-NH₂;
Example 48: H-Apc-D-1Nal-D-1Nal-Phe-Apc-NH₂;
Example 49: H-Apc-D-1Nal-D-2Nal-Phe-Apc-NH₂;
Example 50: H-Apc-D-1Nal-D-1Nal-Phe-Lys-NH₂;
Example 51: H-Apc-D-Bal-D-1Nal-Phe-Apc-NH₂;
Example 52: H-Apc-D-Bal-D-2Nal-Phe-Apc-NH₂;
Example 53: H-Apc-D-Bal-D-1Na1-Phe-Lys-NH₂;
Example 54: H-Apc-D-Bal-D-2Nal-Phe-Lys-NH₂;
Example 55: H-Apc-D-1Nal-D-Trp-2Thi-NH₂;
Example 56: H-Apc-D-Bal-D-Trp-Phe-NH₂;
Example 57: H-Apc-D-1Nal-D-Trp-Taz-NH₂;
Example 58: H-Apc-D-Bal-D-Trp-2Thi-NH₂;
Example 59: H-Apc-D-Bal-D-Trp-Taz-NH₂;
Example 60: H-Apc-D-2Nal-D-Trp-2Thi-NH₂;
Example 61: H-Apc-D-2Nal-D-Trp-Taz-NH₂;
Example 62: H-Inp-D-1Nal-D-Trp-Taz-Apc-NH₂;
Example 63: H-Inp-D-Bal-D-Trp-Taz-Apc-NH₂;
Example 64: H-Apc-D-1Nal-D-Trp-Taz-Apc-NH₂;
Example 65: H-Apc-D-Bal-D-Trp-Taz-Apc-NH₂;
Example 66: H-Inp-D-2Nal-D-Trp(Ψ)-Pim;
Example 67: H-Inp-D-1Nal-D-Trp(Ψ)-Pim;
Example 68: H-Inp-D-Bal-D-Trp(Ψ)-Pim;
Example 69: H-Aib-D-Ser(Bzl)-D-Trp(Ψ)-Pim; and
Example 70: H-Inp-D-Trp-D-2Nal(Ψ)-Pim.

### BRIEF DESCRIPTION OF THE DRAWINGS

FIG. 1A and FIG. 1B show full time course plots of the pharmacokinetic profiles (median values) obtained after a single subcutaneous administration to Sprague-Dawley rats dosed at 2.5 mg/kg body weight, of a formulation comprising 200 mg/mL (20% w/v) of a pamoate salt of Example 1 dissolved in a 50% PEG200 and 50% water (v/v) solvent, on a normal scale and on a logarithmic scale, respectively.

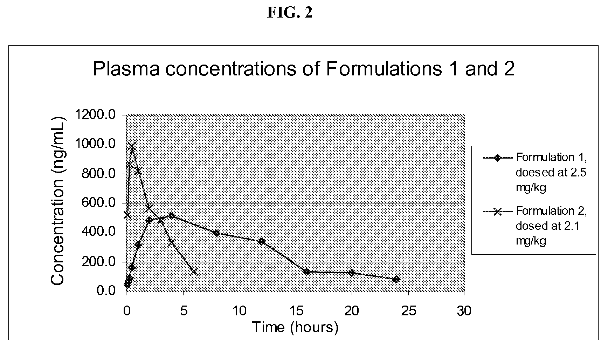

FIG. 2 shows a comparison plot of different formulations of Example 1 dosed by subcutaneous injections.

### DETAILED DESCRIPTION OF THE INVENTION

The nomenclature used to define the peptides herein is that typically used in the art wherein the amino group at the N-terminus appears to the left and the carboxyl group at the C-terminus appears to the right. Where the amino acid has isomeric forms, it is the L form of the amino acid that is represented unless otherwise explicitly indicated. Unless defined otherwise, all technical and scientific terms used herein have the same meaning as commonly understood by one of ordinary skill in the art to which this invention belongs.

Abbreviations used herein are defined as follows:

| | | |
|---|---|---|
| Apc | amino piperidinylcarboxylic acid, *i.e.,* | 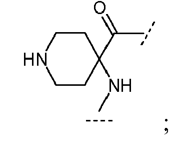 |
| Bal | 3-benzothienylalanine, *i.e.,* | 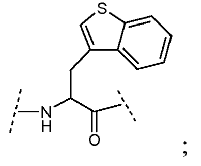 |
| Bip | 4,4'-biphenylalanine, *i.e.*, | 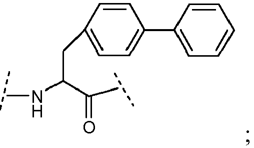 |
| Bpa | 4-benzoylphenylalanine, *i.e.*, | 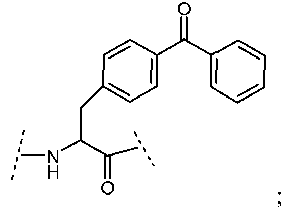 |
| Dip | β,β-diphenylalanine, *i.e.*, | 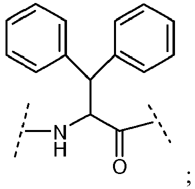 |
| Inp | isonipecotic acid, *i.e.*, | 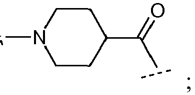 |
| Lys or K | lysine | |
| 1Nal | β-(1-naphthyl)alanine: | |
| 2Nal | β-(2-naphthyl)alanine; | |
| Orn | ornithine | |
| 3Pal | β-(3-pyridyl)-alanine, *i.e.*, | 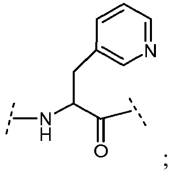 |
| 4Pal | β-(4-pyridyl)-alanine, *i.e.*, | 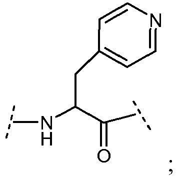 |
| Pff | pentafluorophenylalanine, *i.e.*, | 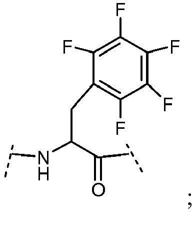 |
| Phe or F | phenylalanine | |
| Pim | 2'-(4-phenyl)imidazolyl, *i.e.*, | 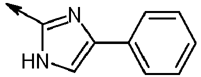 |
| Ser or S | serine | |
| Taz | β-(4-thiazolyl)alanine, *i.e.*, | 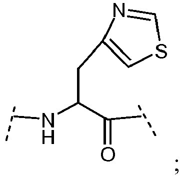 |
| 2Thi | β-(2-thienyl)alanine, *i.e.*, | 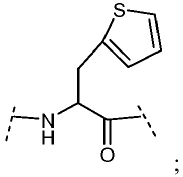 |
| Thr or T | threonine | |
| Trp or W | tryptophan | |

Certain other abbreviations used herein are defined as follows:

| | |
|---|---|
| BSA: | bovine serum albumin |
| DMF: | dimethylformamide |
| HPLC: | high performance liquid chromatography |
| Sodium pamoate: | pamoic acid disodium salt having the structure of |
| | 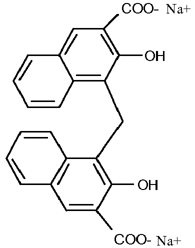 |
| LC-MS: | liquid chromatography mass spectrometry |
| LOQ: | limit of quantification |
| MRM: | multiple reaction monitoring |
| | 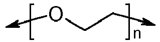 |
| PEG: | poly(ethylene glycol), which has the structure of wherein n an integer between 1 and 2,000 |
| PEG200: | poly(ethylene glycol) with an average molecular weight of about 200 Da |
| PEG400: | poly(ethylene glycol) with an average molecular weight of about 400 Da |
| Tris-HCl: | tris(hydroxymethyl)aminomethane hydrochloride |

Unless otherwise apparent, abbreviations (e.g. Ala) of amino acids in this disclosure stand for the structure of -NH-C(R)(R')-CO-, wherein R and R' each is, independently, hydrogen or the side chain of an amino acid (e.g., R = CH₃ and R' = H for Ala), or R and R' may be joined to form a ring system.

When a non-amino acid imidazole moiety, (e.g., Pim, defined above), is present at the C-terminus of a compound of the invention it is understood that the imidazole moiety is attached to the adjacent amino acid via a pseudo-peptide bond, wherein a bond is formed between the position 2 carbon of the imidazole ring and the alpha carbon of the amino acid. For example, in the case where the adjacent amino acid is D-tryptophan (D-Trp) and the imidazole moiety is Pim, the C-terminus of the peptide would appear as follows: 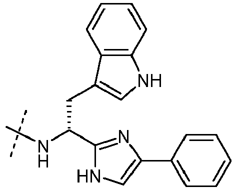 For clarity, in the written formula for such a compound the presence of this bond is indicated by the Greek letter "Ψ" alone in parentheses. For example, the written formula H-Inp-D-Trp-D-2Nal(Ψ)-Pim denotes the structure:

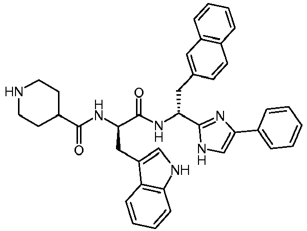

### Synthesis

The peptides of this disclosure can be prepared using the techniques disclosed in WO 2004/014415, at pages 34-42. In addition, examples of techniques for biochemical synthesis involving the introduction of a nucleic acid into a cell and expression of nucleic acids are provided in Ausubel, Current Protocol in Molecular Biology, John Wiley, 1987-1998, and Sambrook et al., in Molecular- Cloning, A Laboratory Manual, 2nd Edition, Cold Spring Harbor Laboratory Press, 1989. Techniques for chemical synthesis of polypeptides are also well known in the art. (See *e.g.,* Vincent in Peptide and Protein Drug Delivery, New York, N.Y., Dekker, 1990.) For example, the peptides of this invention can be prepared by standard solid phase peptide synthesis. (See, *e.g.*, Stewart, J.M., et al., Solid Phase Synthesis (Pierce Chemical Co., 2d ed. 1984)) Physical data for the exemplified peptides are given in Table 1.

**TABLE 1**

| Ex. No. | Sequence | Mol.Wt. (Calc.) | Mol.Wt. (MS-ES) | Purity (%) |
|---|---|---|---|---|
| 1 | H-Inp-D-Bal-D-Trp-Phe-Apc-NH₂ | 790.99 | 790.4 | 97 |
| 2 | H-Inp-D-1Nal-D-Trp-3Pa1-Lys-NH₂ | 787.96 | 787.4 | 96 |
| 3 | H-Inp-D-2Nal-D-Trp-4Pal-Lys-NH₂ | 787.96 | 787.4 | 99 |
| 4 | H-Inp-D-2Nal-D-Trp-Orn-Lys-NH₂ | 753.94 | 753.4 | 98 |
| 5 | H-Inp-D-Bip-D-Trp-Phe-Lys-NH₂ | 813.01 | 812.4 | 99 |
| 6 | H-Inp-D-2Nal-D-Trp-Thr(Bzl)-Lys-NH₂ | 831.03 | 830.4 | 98 |
| 7 | H-Inp-D-2Nal-D-Trp-Pff-Lys-NH₂ | 876.92 | 876.3 | 98 |
| 8 | H-Inp-D-2Nal-D-Trp-Thi-Lys-NH₂ | 793.00 | 792.4 | 98 |
| 9 | H-Inp-D-2Nal-D-Trp-Taz-Lys-NH₂ | 793.99 | 793.4 | 97 |
| 10 | H-Inp-D-Dip-D-Trp-Phe-Lys-NH₂ | 813.01 | 812.4 | 98 |
| 11 | H-Inp-D-Bpa-D-Trp-Phe-Lys-NH₂ | 841.02 | 840.4 | 95 |
| 12 | H-Inp-D-2Nal-D-Bpa-Phe-Lys-NH₂ | 852.04 | 851.3 | 99 |
| 13 | H-Inp-D-2Nal-D-Trp-3Pal-NH₂ | 659.79 | 659.3 | 99 |
| 14 | H-Inp-D-2Nal-D-Trp-4Pal-NH₂ | 659.79 | 659.3 | 98 |
| 15 | H-Inp-D-1Nal-D-Trp-3Pal-NH₂ | 659.79 | 659.3 | 98 |
| 16 | H-Inp-D-Bip-D-Trp-Phe-NH₂ | 684.84 | 684.3 | 99 |
| 17 | H-Inp-D-2Nal-D-Trp-Thr(Bzl)-NH₂ | 702.85 | 702.3 | 99 |
| 18 | H-Inp-D-2Nal-D-Trp-Pff-NH₂ | 748.75 | 748.2 | 99 |
| 19 | H-Inp-D-2Nal-D-Trp-2Thi-NH₂ | 664.83 | 664.2 | 99 |
| 20 | H-Inp-D-2Nal-D-Trp-Taz-NH₂ | 665.82 | 665.3 | 98 |
| 21 | H-Inp-D-Dip-D-Trp-Phe-NH₂ | 684.84 | 684.3 | 98 |
| 22 | H-Inp-D-2Nal-D-Dip-Phe-NH₂ | 695.86 | 695.3 | 99 |
| 23 | H-Inp-D-Bal-D-Trp-Phe-NH₂ | 664.83 | 664.3 | 97 |
| 24 | H-Inp-D-2Nal-D-Bal-Phe-NH₂ | 675.85 | 675.2 | 99 |
| 25 | H-Inp-D-2Nal-D-Trp-3Pal-Lys-NH2 | 787.96 | 787.5 | 97 |
| 26 | H-Inp-D-Bal-D-Trp-2Thi-Lys-NH₂ | 799.03 | 798.4 | 99 |
| 27 | H-Inp-D-Bal-D-Trp-Phe-Lys-NH2 | 793.00 | 792.4 | 99 |
| 28 | H-Inp-D-1Nal-D-Trp-2Thi-Lys-NH₂ | 793.00 | 792.4 | 99 |
| 29 | H-Inp-D-2Nal-D-Trp-Phe-Apc-NH₂ | 784.96 | 784.4 | 98 |
| 30 | H-Inp-D-1Nal-D-Trp-Phe-Apc-NH₂ | 784.96 | 784.4 | 98 |
| 31 | H-Apc-D-2Nal-D-Trp-Phe-Lys-NH₂ | 801.99 | 801.4 | 98 |
| 32 | H-Apc-D-1Nal-D-Trp-2Thi-Lys-NH₂ | 808.02 | 807.4 | 99 |
| 33 | H-Inp-D-1Nal-D-Trp-2Thi-NH₂ | 664.83 | 664.2 | 98 |
| 34 | H-Apc-D-1Nal-D-Trp-Phe-NH₂ | 673.81 | 673.3 | 99 |
| 35 | H-Inp-D-1Nal-D-Trp-Taz-Lys-NH₂ | 793.99 | 793.5 | 99 |
| 36 | H-Inp-D-Bal-D-Trp-Taz-Lys-NH₂ | 800.02 | 799.4 | 99 |
| 37 | H-Apc-D-1Nal-D-Trp-Taz-Lys-NH₂ | 809.00 | 808.5 | 99 |
| 38 | H-Apc-D-Bal-D-Trp-Taz-Lys-NH₂ | 815.03 | 814.4 | 99 |
| 39 | H-Apc-D-Bal-D-Trp-2Thi-Lys-NH₂ | 814.04 | 813.4 | 98 |
| 40 | H-Inp-D-1Nal-D-Trp-2Thi-Apc-NH₂ | 790.99 | 790.5 | 97 |
| 41 | H-Inp-D-Bal-D-Trp-2Thi-Apc-NH₂ | 797.01 | 796.4 | 97 |
| 42 | H-Apc-D-1Nal-D-Trp-2Thi-Apc-NH₂ | 806.00 | 805.5 | 97 |
| 43 | H-Apc-D-Bal-D-Trp-2Thi-Apc-NH₂ | 812.03 | 811.4 | 98 |
| 44 | H-Apc-D-1Nal-D-Trp-Phe-Lys-NH₂ | 801.99 | 801.5 | 98 |
| 45 | H-Apc-D-Bal-D-Trp-Phe-Lys-NH₂ | 808.02 | 807.5 | 99 |
| 46 | H-Apc-D-1Nal-D-Trp-Phe-Apc-NH₂ | 799.97 | 799.5 | 98 |
| 47 | H-Apc-D-Bal-D-Trp-Phe-Apc-NH₂ | 806.00 | 805.5 | 98 |
| 48 | H-Apc-D-1Nal-D-1Nal-Phe-Apc-NH₂ | 811.00 | 810.5 | 95 |
| 49 | H-Apc-D-1Nal-D-2Nal-Phe-Apc-NH₂ | 811.00 | 810.5 | 96 |
| 50 | H-Apc-D-1Nal-D-1Nal-Phe-Lys-NH₂ | 813.01 | 812.5 | 99 |
| 51 | H-Apc-D-Bal-D-1Nal-Phe-Apc-NH₂ | 817.02 | 816.5 | 96 |
| 52 | H-Apc-D-Bal-D-2Nal-Phe-Apc-NH₂ | 817.02 | 816.5 | 94 |
| 53 | H-Apc-D-Bal-D-1Nal-Phe-Lys-NH₂ | 819.04 | 818.5 | 99 |
| 54 | H-Apc-D-Bal-D-2Nal-Phe-Lys-NH₂ | 819.04 | 818.5 | 98 |
| 55 | H-Apc-D-1Nal-D-Trp-2Thi-NH₂ | 679.84 | 679.2 | 98 |
| 56 | H-Apc-D-Bal-D-Trp-Phe-NH₂ | 679.84 | 679.3 | 99 |
| 57 | H-Apc-D-1Nal-D-Trp-Taz-NH₂ | 680.83 | 680.3 | 99 |
| 58 | H-Apc-D-Bal-D-Trp-2Thi-NH₂ | 685.87 | 685.2 | 97 |
| 59 | H-Apc-D-Bal-D-Trp-Taz-NH₂ | 686.86 | 686.2 | 99 |
| 60 | H-Apc-D-2Nal-D-Trp-2Thi-NH₂ | 679.84 | 679.2 | 95 |
| 61 | H-Apc-D-2Nal-D-Trp-Taz-NH2 | 680.83 | 680.2 | 97 |
| 62 | H-Inp-D-1Nal-D-Trp-Taz-Apc-NH₂ | 791.97 | 791.5 | 98 |
| 63 | H-Inp-D-Bal-D-Trp-Taz-Apc-NH₂ | 798.00 | 797.4 | 99 |
| 64 | H-Apc-D-1Nal-D-Trp-Taz-Apc-NH₂ | 806.99 | 806.5 | 99 |
| 65 | H-Apc-D-Bal-D-Trp-Taz-Apc-NH₂ | 813.02 | 812.4 | 98 |
| 66 | H-Inp-D-2Nal-D-Trp(Ψ)-Pim | 610.77 | 611.4 | 99 |
| 67 | H-Inp-D-1Nal-D-Trp(Ψ)-Pim | 610.77 | 611.3 | 99 |
| 68 | H-Inp-D-Bal-D-Trp(Ψ)-Pim | 616.79 | 617.3 | 99 |
| 69 | H-Aib-D-Ser(Bzl)-D-Trp(Ψ)-Pim | 564.69 | 565.3 | 99 |
| 70 | H-Inp-D-Trp-D-2Nal(Ψ)-Pim | 610.77 | 611.4 | 99 |

### • Preparation of Pamoate Salt of Example 1

The acetate salt of Example 1 (200 mg, 0.22 mmole) was dissolved in 10 mL of water. Sodium pamoate (190 mg, 0.44 mmole) was dissolved in 10 mL of water. The two solutions were combined and mixed well. The precipitates were collected by centrifugation at 3000 rpm for 20 minutes, washed for three times with water, and dried by lyophilization.

### In Vitro Studies

Compounds of the present invention can be and were tested for activity as ligands of the GHS receptor according to the following procedures. One skilled in the art would know that procedures similar to those described herein may be used to assay the binding activities of the compounds of the invention to melanocortin receptor molecules.

### • Radioligand Binding Assays

Cellular membranes used for the *in vitro* receptor binding assay were obtained from transgenic CHO-K1 cells stably expressing the human recombinant GHS receptor. CHO-K1 cells stably expressing the hGHS receptor were homogenized in 20 ml of ice-cold 50 mM Tris-HCl with a Brinkman Polytron (Westbury, NY, USA) (setting 6, 15 sec). The homogenates were washed twice by centrifugation (39,000 g / 10 min), and the final pellets were resuspended in 50 mM Tris-HCl, containing 2.5 mM MgCl₂, and 0.1% BSA. For assay, aliquots (0.4 ml) were incubated with 0.05 nM (¹²⁵I)ghrelin (~2000 Ci/mmol, Perkin Elmer Life Sciences, Boston, MA, USA), with and without 0.05 ml of unlabeled competing test compounds of the invention. After a 60 min incubation (4°C), the bound (¹²⁵I)ghrelin was separated from the free by rapid filtration through GF/C filters (Brandel, Gaithersburg, MD, USA), which had been previously soaked in 0.5% polyethyleneimine/0.1% BSA. The filters were then washed three times with 5-ml aliquots of ice-cold 50 mM Tris-HCl and 0.1% bovine serum albumin, and the bound radioactivity trapped on the filters was counted by gamma spectrometry (Wallac LKB, Gaithersburg, MD, USA). Specific binding was defined as the total (¹²⁵I)ghrelin bound minus that bound in the presence of 1000 nM ghrelin (Bachem, Torrence, CA, USA). Specific binding data for the exemplified peptides are given in Table 2.

**TABLE 2**

| Ex. No. | Sequence | hGHS Ki (nM) |
|---|---|---|
| 1 | H-Inp-D-Bal-D-Trp-Phe-Apc-NH₂ | 0.42 |
| 2 | H-Inp-D-1Nal-D-Trp-3Pal-Lys-NH₂ | 1.05 |
| 3 | H-Inp-D-2Nal-D-Trp-4Pal-Lys-NH₂ | 7.35 |
| 4 | H-Inp-D-2Nal-D-Trp-Orn-Lys-NH₂ | 243.00 |
| 5 | H-Inp-D-Bip-D-Trp-Phe-Lys-NH₂ | 1.35 |
| 6 | H-Inp-D-2Nal-D-Trp-Thr(Bzl)-Lys-NH₂ | 1.55 |
| 7 | H-Inp-D-2Nal-D-Trp-Pff-Lys-NH₂ | 25.43 |
| 8 | H-Inp-D-2Nal-D-Trp-2Thi-Lys-NH₂ | 0.45 |
| 9 | H-Inp-D-2Nal-D-Trp-Taz-Lys-NH₂ | 0.80 |
| 10 | H-Inp-D-Dip-D-Trp-Phe-Lys-NH₂ | 46.78 |
| 11 | H-Inp-D-Bpa-D-Trp-Phe-Lys-NH₂ | 93.75 |
| 12 | H-Inp-D-2Nal-D-Bpa-Phe-Lys-NH₂ | 419.00 |
| 13 | H-Inp-D-2Nal-D-Trp-3Pal-NH₂ | 11.35 |
| 14 | H-Inp-D-2Nal-D-Trp-4Pal-NH₂ | 113.50 |
| 15 | H-Inp-D-1Nal-D-Trp-3Pal-NH₂ | 16.10 |
| 16 | H-Inp-D-Bip-D-Trp-Phe-NH₂ | 20.00 |
| 17 | H-Inp-D-2Nal-D-Trp-Thr(Bzl)-NH₂ | 4.46 |
| 18 | H-Inp-D-2Nal-D-Trp-Pff-NH₂ | 36.31 |
| 19 | H-Inp-D-2Nal-D-Trp-2Thi-NH₂ | 4.11 |
| 20 | H-Inp-D-2Nal-D-Trp-Taz-NH₂ | 6.17 |
| 21 | H-Inp-D-Dip-D-Trp-Phe-NH₂ | 104.83 |
| 22 | H-Inp-D-2Nal-D-Dip-Phe-NH₂ | 104.80 |
| 23 | H-Inp-D-Bal-D-Trp-Phe-NH₂ | 2.30 |
| 24 | H-Inp-D-2Nal-D-Bal-Phe-NH₂ | 27.40 |
| 25 | H-Inp-D-2Nal-D-Trp-3Pal-Lys-NH₂ | 1.58 |
| 26 | H-Inp-D-Bal-D-Trp-2Thi-Lys-NH₂ | 0.42 |
| 27 | H-Inp-D-Bal-D-Trp-Phe-Lys-NH₂ | 0.33 |
| 28 | H-Inp-D-1Nal-D-Trp-2Thi-Lys-NH₂ | 0.31 |
| 29 | H-Inp-D-2Nal-D-Trp-Phe-Apc-NH₂ | 0.64 |
| 30 | H-Inp-D-1Nal-D-Trp-Phe-Apc-NH₂ | 0.36 |
| 31 | H-Apc-D-2Nal-D-Trp-Phe-Lys-NH₂ | 0.42 |
| 32 | H-Apc-D-1Nal-D-Trp-2Thi-Lys-NH₂ | 0.29 |
| 33 | H-Inp-D-1Nal-D-Trp-2Thi-NH₂ | 0.87 |
| 34 | H-Apc-D-1Nal-D-Trp-Phe-NH₂ | 0.70 |
| 35 | H-Inp-D-1Nal-D-Trp-Taz-Lys-NH₂ | 1.11 |
| 36 | H-Inp-D-Bal-D-Trp-Taz-Lys-NH₂ | 0.52 |
| 37 | H-Apc-D-1Nal-D-Trp-Taz-Lys-NH₂ | 0.45 |
| 38 | H-Apc-D-Bal-D-Trp-Taz-Lys-NH₂ | 0.50 |
| 39 | H-Apc-D-Bal-D-Trp-2Thi-Lys-NH₂ | 0.36 |
| 40 | H-Inp-D-1Nal-D-Trp-2Thi-Apc-NH₂ | 0.53 |
| 41 | H-Inp-D-Bal-D-Trp-2Thi-Apc-NH₂ | 0.40 |
| 42 | H-Apc-D-1Nal-D-Trp-2Thi-Apc-NH₂ | 0.46 |
| 43 | H-Apc-D-Bal-D-Trp-2Thi-Apc-NH₂ | 0.51 |
| 44 | H-Apc-D-1Nal-D-Trp-Phe-Lys-NH₂ | 0.42 |
| 45 | H-Apc-D-Bal-D-Trp-Phe-Lys-NH₂ | 0.32 |
| 46 | H-Apc-D-1Nal-D-Trp-Phe-Apc-NH₂ | 0.46 |
| 47 | H-Apc-D-Bal-D-Trp-Phe-Apc-NH₂ | 0.71 |
| 48 | H-Apc-D-1Nal-D-1Nal-Phe-Apc-NH₂ | 1.99 |
| 49 | H-Apc-D-1Nal-D-2Nal-Phe-Apc-NH₂ | 1.71 |
| 50 | H-Apc-D-1Nal-D-1Nal-Phe-Lys-NH₂ | 1.32 |
| 51 | H-Apc-D-Bal-D-1Nal-Phe-Apc-NH₂ | 3.48 |
| 52 | H-Apc-D-Bal-D-2Nal-Phe-Apc-NH₂ | 1.49 |
| 53 | H-Apc-D-Bal-D-1Nal-Phe-Lys-NH₂ | 1.46 |
| 54 | H-Apc-D-Bal-D-2Nal-Phe-Lys-NH₂ | 0.68 |
| 55 | H-Apc-D-1Nal-D-Trp-2Thi-NH₂ | 0.73 |
| 56 | H-Apc-D-Bal-D-Trp-Phe-NH₂ | 0.89 |
| 57 | H-Apc-D-1Nal-D-Trp-Taz-NH₂ | 1.41 |
| 58 | H-Apc-D-Bal-D-Trp-2Thi-NH₂ | 0.98 |
| 59 | H-Apc-D-Bal-D-Trp-Taz-NH₂ | 1.62 |
| 60 | H-Apc-D-2Nal-D-Trp-2Thi-NH₂ | 0.95 |
| 61 | H-Apc-D-2Nal-D-Trp-Taz-NH₂ | 2.11 |
| 62 | H-Inp-D-1Nal-D-Trp-Taz-Apc-NH₂ | 1.19 |
| 63 | H-Inp-D-Bal-D-Trp-Taz-Apc-NH₂ | 0.83 |
| 64 | H-Apc-D-1Nal-D-Trp-Taz-Apc-NH₂ | 0.98 |
| 65 | H-Apc-D-Bal-D-Trp-Taz-Apc-NH₂ | 1.13 |
| 66 | H-Inp-D-2Nal-D-Trp(Ψ)-Pim | 116.68 |
| 67 | H-Inp-D-1Nal-D-Trp(Ψ)-Pim | 50.55 |
| 68 | H-Inp-D-Bal-D-Trp(Ψ)-Pim | 48.73 |
| 69 | H-Aib-D-Ser(Bzl)-D-Trp(Ψ)-Pim | 753.33 |
| 70 | H-Inp-D-Trp-D-2Nal(Ψ)-Pim | 182.00 |

### • Solubility Study

Pamoate salt of Example 1 (50 mg) was weighted into a microcentrifuge tube, and 125 µL of PEG200 and 125 µL of water were added thereafter. The mixture was sonicated to facilitate dissolution. A clear solution was obtained.

The solubility of pamoate salt of Example 1 was determined by mixing the peptide in water or PBS, followed by HPLC determination of the concentration in the supernatant, and the results are shown in Table 3.

**TABLE 3**

| Solubility of | Water, pH 7.0 | PBS, pH 7.4 |
|---|---|---|
| Pamoate salt of Example 1 | 0.06 mg/mL | 0.07 mg/mL |
| Pamoate salt of Example 1 with Zn | 0.14 mg/mL | 0.08 mg/mL |

### • Pharmacokinetic Studies of Formulations of Example 1

"Formulation 1" of Example 1 was prepared by dissolving a pamoate salt of Example 1 in a 50% PEG200 and 50% water (v/v) solution, at a concentration of 200 mg/mL (20% w/v).
"Formulation 2" of Example 1 was prepared by dissolving an acetate salt of Example 1 in a saline/2% heat inactivated mouse serum/5% DMA/2% tween-80 solution.

### • Dosing

For Formulation 1, Sprague-Dawley rats were dosed via subcutaneous injection at a fixed amount of either 5 µL/rat or 1.0 mg/rat, or a variable amount of roughly 2.5 mg/kg body weight.
For Formulation 2, Sprague-Dawley rats were dosed via subcutaneous injection at a variable amount of 2.1 mg/kg body weight.

### • Sample Preparation

For Formulations 1 and 2, 50 µL of plasma was acidified with 2.5 µL of formic acid and precipitated with 150 µL of acetonitrile. The supernatant was collected by centrifugation. 50 µL of the preparation was injected for LC-MS/MS analysis.

### • LC-MS/MS Analysis

For Formulations 1 and 2, LC-MS/MS analysis was performed with an API4000 mass spectrometer system equipped with a Turbo Ionspray probe. The MRM mode of molecular ion detection with an ion pair of 396.5 / 112.3 was used. HPLC separation was performed with a Luna C8(2) 2x30 mm 3µ column run from 0% B to 80% B in 10 minutes at a flow rate of 0.3 mL/minute. Buffer A is 1% formic acid in water and buffer B is 1% formic acid in acetonitrile. LOQ was 5 ng/mL.

### • Results and Summary

The plasma concentrations of Example 1, dosed with Formulation 1, were calculated with its standard calibration plot and the results are shown in Table 4.

**TABLE 4**

| Time | Plasma concentration (ng/mL) of Example 1, dosed with Formulation 1 | Plasma concentration (ng/mL) of Example 1, dosed with Formulation 2 |
|---|---|---|
| 5 minutes | 47.0 | 520 |
| 10 minutes | 62.4 | N/A |
| 15 minutes | 85.4 | 860 |
| 30 minutes | 162.5 | 990 |
| 1 hour | 312.5 | 820 |
| 2 hours | 485.0 | 560 |
| 3 hours | N/A | 480 |
| 4 hours | 509.5 | 330 |
| 6 hours | N/A | 130 |
| 8 hours | 396.0 | 0 |
| 12 hours | 334.0 | 0 |
| 16 hours | 132.7 | 0 |
| 20 hours | 121.0 | 0 |
| 24 hours | 84.0 | 0 |

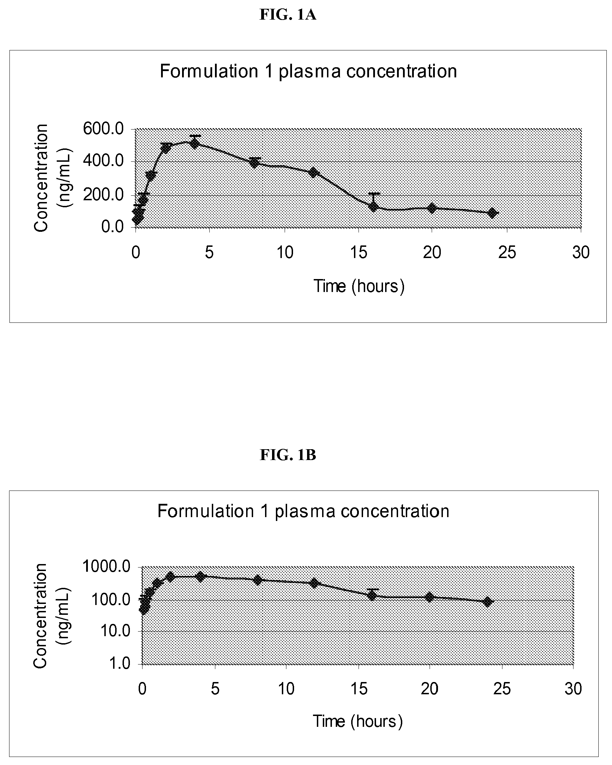

Full time course plots of the pharmacokinetic profiles of Formulation 1 are shown on a normal scale in FIG. 1A, and on a logarithmic scale in FIG. 1B.

Some pharmacokinetic parameters of Example 1, dosed with Formulation 1, are shown in Table 5.

**TABLE 5**

| | Example 1 dosed with Formulation 1 | Example 1 dosed with Formulation 2 |
|---|---|---|
| Tₘₐₓ (hours) | 4 | 0.5 |
| Cₘₐₓ (ng/mL) | 543 | 990 |
| AUC (ng-hr/mL) | 7493 | 3239 |
| CL (ml/hour) | 333 | 725 |
| T_{1/2} (hours) | 6.7 | 1.8 |

The results indicate that the formulations of Example 1 according to the present invention as described herein provide for acceptable sustained release formulations with improved pharmacokinetic parameters and flatter release profiles which may result in attenuated side effects and improved efficacy. For instance, Formulation 1 is shown to have a release profile of greater than 24 hours after a single subcutaneous injection, with significantly low Cₘₐₓ and significantly long Tₘₐₓ. Moreover, the pamoate salt formulation of Example 1, *i.e.,* Formulation 1, is shown to have significantly increased T_{1/2} compared to the acetate salt formulation of Example 1, *i.e.,* Formulation 2.

Additional embodiments of the present invention will be apparent from the foregoing disclosure and are intended to be encompassed by the invention as described fully herein and defined in the following claims.

## Claims

1. A pharmaceutical composition of a clear solution, comprising a peptide that acts as a ligand of the GHS receptor, wherein the peptide is H-Inp-D-Bal-D-Trp-Phe-Apc-NH₂, or a pharmaceutically acceptable salt thereof, in which the peptide forms an *in situ* depot after subcutaneous or intramuscular administration to a subject, wherein the pharmaceutical composition further comprises PEG, wherein said peptide is in a pamoate salt form, and wherein said clear solution is an aqueous solution.

2. The pharmaceutical composition according to claim 1, wherein said PEG has an average molecular weight of from about 200 to about 10,000.

3. The pharmaceutical composition according to claim 2, wherein said peptide is dissolved in a PEG200 or PEG400 aqueous solution, in which the volume-to-volume ratio of PEG to water is from about 1:9 to about 1:1.

4. The pharmaceutical composition according to any one of the preceding claims, wherein the weight-to-volume concentration of said peptide is between about 0.1 mg/mL and about 2000 mg/mL, and/or wherein the pH of said composition is between about 3.0 and about 8.0.

5. The pharmaceutical composition according to claim 4, wherein said pamoate salt of H-Inp-D-Bal-D-Trp-Phe-Apc-NH₂ is dissolved:
(a) in a PEG400/aqueous solution, in which the volume-to-volume ratio of PEG400 to water is about 1:1, and in which the weight-to-volume concentration of the peptide is about 200 mg/mL;
(b) in a PEG200/aqueous solution, in which the volume-to-volume ratio of PEG200 to water is about 1:1, and in which the weight-to-volume concentration of the peptide is about 200 mg/mL;
(c) in a PEG400/PBS solution, in which the volume-to-volume ratio of PEG400 to PBS is about 1:1, and in which the weight-to-volume concentration of the peptide is about 300 mg/mL; or
(d) in a PEG400 saline solution, in which the volume-to-volume ratio of PEG400 to saline solution is about 1:1, and in which the weight-to-volume concentration of the peptide is about 300 mg/mL.

6. The pharmaceutical composition according to any one of the preceding claims further comprising a divalent metal.

7. The pharmaceutical composition according to claim 6, wherein said divalent metal is zinc.

8. The pharmaceutical composition according to any one of the preceding claims further comprising: a preservative; an isotonic agent; a stabilizer; a surfactant; a chelating agent; and/or a buffer.

9. The pharmaceutical composition according to claim 8:
(a) wherein said preservative is selected from the group consisting of m-cresol, phenol, benzyl alcohol, and methyl paraben;
(b) wherein said preservative is present in a concentration from about 0.01 mg/mL to about 100 mg/mL;
(c) wherein said isotonic agent is present in a concentration from about 0.01 mg/mL to about 100 mg/mL;
(d) wherein said stabilizer is selected from the group consisting of imidazole, arginine and histidine; and
(e) wherein said buffer is selected from the group consisting of Tris, ammonium acetate, sodium acetate, glycine, aspartic acid, and Bis-Tris.

## Patentansprüche

1. Pharmazeutische Zusammensetzung einer klaren Lösung, umfassend ein Peptid, das als Ligand des GHS-Rezeptors agiert, wobei es sich bei dem Peptid um H-Inp-D-Bal-D-Trp-Phe-Apc-NH₂ oder ein pharmazeutisch unbedenkliches Salz davon handelt, wobei das Peptid nach subkutaner oder intramuskulärer Verabreichung an ein Subjekt ein *in situ*-Depot bildet, wobei die pharmazeutische Zusammensetzung weiter PEG umfasst, wobei das Peptid die Form eines Pamoatsalzes aufweist, und wobei es sich bei der klaren Lösung um eine wässrige Lösung handelt.

2. Pharmazeutische Zusammensetzung nach Anspruch 1, wobei das PEG ein durchschnittliches Molekulargewicht von etwa 200 bis etwa 10.000 aufweist.

3. Pharmazeutische Zusammensetzung nach Anspruch 2, wobei das Peptid in einer wässrigen PEG200- oder PEG400-Lösung aufgelöst ist, in der das Verhältnis von Volumen zu Volumen von PEG zu Wasser etwa 1:9 bis etwa 1:1 beträgt.

4. Pharmazeutische Zusammensetzung nach einem der vorhergehenden Ansprüche, wobei die Konzentration von Gewicht zu Volumen des Peptids etwa 0,1 mg/ml bis etwa 2000 mg/ml beträgt, und/oder wobei der pH-Wert der Zusammensetzung etwa 3,0 bis etwa 8,0 beträgt.

5. Pharmazeutische Zusammensetzung nach Anspruch 4, wobei das Pamoatsalz von H-Inp-D-Bal-D-Trp-Phe-Apc-NH₂ aufgelöst ist:
(a) in einer PEG400-/wässrigen Lösung, in der das Verhältnis von Volumen zu Volumen von PEG400 zu Wasser etwa 1:1 beträgt, und in der die Konzentration von Gewicht zu Volumen des Peptids etwa 200 mg/ml beträgt;
(b) in einer PEG200-/wässrigen Lösung, in der das Verhältnis von Volumen zu Volumen von PEG200 zu Wasser etwa 1:1 beträgt, und in der die Konzentration von Gewicht zu Volumen des Peptids etwa 200 mg/ml beträgt;
(c) in einer PEG400-/PBS-Lösung, in der das Verhältnis von Volumen zu Volumen von PEG400 zu PBS etwa 1:1 beträgt, und in der die Konzentration von Gewicht zu Volumen des Peptids etwa 300 mg/ml beträgt; oder
(d) in einer PEG400-/Salzlösung, in der das Verhältnis von Volumen zu Volumen von PEG400 zu Salzlösung etwa 1:1 beträgt, und in der die Konzentration von Gewicht zu Volumen des Peptids etwa 300 mg/ml beträgt;

6. Pharmazeutische Zusammensetzung nach einem der vorhergehenden Ansprüche, weiter umfassend ein zweiwertiges Metall.

7. Pharmazeutische Zusammensetzung nach Anspruch 6, wobei es sich bei dem zweiwertigen Metall um Zink handelt.

8. Pharmazeutische Zusammensetzung nach einem der vorhergehenden Ansprüche, weiter umfassend: ein Konservierungsmittel; ein isotonisches Mittel; ein Stabilisierungsmittel; ein oberflächenaktives Mittel; ein Chelatisierungsmittel; und/oder einen Puffer.

9. Pharmazeutische Zusammensetzung nach Anspruch 8:
(a) wobei das Konservierungsmittel aus der aus m-Kresol, Phenol, Benzylalkohol und Methylparaben bestehenden Gruppe ausgewählt ist;
(b) wobei das Konservierungsmittel in einer Konzentration von etwa 0,01 mg/ml bis etwa 100 mg/ml vorhanden ist;
(c) wobei das isotonische Mittel in einer Konzentration von etwa 0,01 mg/ml bis etwa 100 mg/ml vorhanden ist;
(d) wobei das Stabilisierungsmittel aus der aus Imidazol, Arginin und Histidin bestehenden Gruppe ausgewählt ist; und
(e) wobei der Puffer aus der aus Tris, Ammoniumacetat, Natriumacetat, Glycin, Asparaginsäure und Bis-Tris bestehenden Gruppe ausgewählt ist.

## Revendications

1. Composition pharmaceutique d'une solution transparente, comprenant un peptide faisant office de ligand du récepteur du GHS, dans laquelle le peptide est H-Inp-D-Bal-D-Trp-Phe-Apc-NH₂, ou un sel pharmaceutiquement acceptable de celui-ci, dans laquelle le peptide forme un dépôt *in situ* après administration sous-cutanée ou intramusculaire à un sujet, dans laquelle la composition pharmaceutique comprend en outre PEG, dans laquelle ledit peptide est sous forme de sel de pamoate, et dans laquelle ladite solution transparente est une solution aqueuse.

2. Composition pharmaceutique selon la revendication 1, dans laquelle ledit PEG a un poids moléculaire moyen d'environ 200 à environ 10.000.

3. Composition pharmaceutique selon la revendication 2, dans laquelle ledit peptide est dissous dans une solution aqueuse PEG200 ou PEG400, dans laquelle le rapport volume/volume de PEG à l'eau est d'environ 1:9 à environ 1:1.

4. Composition pharmaceutique selon l'une des revendications précédentes, dans laquelle la concentration poids/volume dudit peptide est comprise entre environ 0,1 mg/mL et environ 2000 mg/mL, et/ou dans laquelle le pH de cette composition se situe entre environ 3,0 et environ 8.0.

5. Composition pharmaceutique selon la revendication 4, dans laquelle ledit sel de pamoate de H-Inp-D-Bal-D-Trp-Phe-Apc-NH₂ est dissoute:
(a) dans une solution PEG400/aqueuse, dans laquelle le rapport volume/volume de PEG400 à l'eau est d'environ 1:1, et dans laquelle la concentration poids/volume du peptide est d'environ 200 mg/mL;
(b) dans une solution PEG200/aqueuse, dans laquelle le rapport volume/volume de PEG200 à l'eau est d'environ 1:1, et dans laquelle la concentration poids/volume du peptide est d'environ 200 mg/mL;
(c) dans une solution PEG400/PBS, dans laquelle le rapport volume/volume de PEG400 au PBS est d'environ 1:1, et dans laquelle la concentration poids/volume du peptide est d'environ 300 mg/mL;ou
(d) dans une solution saline PEG400, dans laquelle le rapport volume/volume de PEG400 à la solution saline est d'environ 1:1, et dans laquelle la concentration poids/volume du peptide est d'environ 300 mg/mL.

6. Composition pharmaceutique selon l'une des revendications précédentes comprenant en outre un métal divalent.

7. Composition pharmaceutique selon la revendication 6, dans laquelle le métal divalent est le zinc.

8. Composition pharmaceutique selon l'une des revendications précédentes comprenant en outre: un conservateur; un agent isotonique, un stabilisateur; un tensio-actif; un agent de chélation; et/ou un tampon.

9. Composition pharmaceutique selon la revendication 8,
(a) dans laquelle ledit conservateur est choisi dans le groupe composé de m-crésol, phénol, alcool benzylique et méthyle paraben;
(b) dans laquelle ledit conservateur est présent à une concentration d'environ 0,01 mg/mL à environ 100 mg/mL;
(c) dans laquelle ledit agent isotonique est présent à une concentration d'environ 0,01 mg/mL à environ 100 mg/mL;
(d) dans lequel ledit stabilisateur est choisi dans le groupe composé de imidazole, d'arginine et d'histidine; et
(e) dans laquelle ledit tampon est choisi dans le groupe composé de Tris, d'acétate d'ammonium, d'acétate de sodium, de glycine, d'acide aspartique et de Bis-Tris.
